# EUROPEAN PATENT APPLICATION

(11) **EP 4 710 967 A1**
(43) Date of publication of application: **18.03.2026**
(21) Application number: 24199811.1
(22) Date of filing: 11.09.2024
(51) Int. Cl.: A61M 5/20, A61M 5/32, A61M 5/315, A61M 5/24

(54) **AUTOINJECTOR WITH DUAL CHAMBER CARTRIDGE**

(71) Applicant: medmix Switzerland AG, 9469 Haag (Rheintal) (CH)
(72) Inventor: Jones, Matthew Meredith, Warwick, CV34 4AB (GB); Flanagan, Oliver Jack, Warwick, CV34 4AB (GB)
(74) Representative: Manitz Finsterwald Patent- und Rechtsanwaltspartnerschaft mbB

(57) **Abstract**

The invention relates to an autoinjector comprising a dual chamber cartridge.

## Description

The invention relates to an autoinjector comprising a dual chamber cartridge.

In various fields of applications such as for example the industrial, the medical and/or the dental sector it is already common practice to provide dispensers or injectors that are capable of storing two or more components separately from one another and of mixing said components prior to said components being dispensed.

It has been found that this approach can also be favourable in the field of (self)application of certain medicaments by medical amateurs as certain medicaments consisting of two or more components which react with one another. However, often the storage time for such medicaments can be enhanced drastically when the respective components are stored separately from one another such that they cannot interact with each other.

The crux with medical amateurs is that overcoming the bar for self-administering a medicament is often not easy. This can often lead to errors in application or even that sterility is broken which consequently then leads to even bigger issues.

This issue can be solved by providing (auto)injectors that are already prefilled with the right amount of each component of a certain medicament and that are configured to mix said components together before said mixture is then (self)injected by the user.

However, contrary to the dispensers used in the industrial sector for instance, it is necessary for (auto)injectors being used for medicaments to provide a sterile environment where the two or more components can be mixed with one another before the dispense of the mixed medicament as well as a sterile environment for the cannula with which said medicament is supposed to be injected.

Therefore, it is an object of the invention to provide an autoinjector with which all of the above issues can be overcome.

The autoinjector according to the invention comprises a dual chamber cartridge comprising two elements of a medicament that are each stored in a separate chamber of the dual chamber cartridge. Said dual chamber cartridge also comprises a bypass passage. The autoinjector further comprises at least one security mechanism configured to secure the autoinjector against premature mixing of the elements and dispensing of said mixed elements.

That is, according to the invention an autoinjector is provided that comprises a dual chamber cartridge for two elements of a medicament that is configured to store each element separately from one another. Furthermore, said dual chamber cartridge comprises a bypass passage that may be configured to enable the two elements to be mixed with one another when a mixing mechanism is triggered.

Furthermore, the autoinjector according to the invention comprises at least one security mechanism that is configured to secure the autoinjector against premature mixing of the elements and dispensing of said mixed elements. In this connection it should further be noted that the security mechanism according to the invention can further be configured to provide a sterile environment for the elements, and in particular a cannula of said autoinjector, prior to mixing the elements respectively dispensing said mixture, i.e. injecting the medicament into a patient. The security mechanism may additionally be configured to protect a user from being injured by the autoinjector by for example shielding a cannula from the environment when the autoinjector is in a storage state.

Hence, with the autoinjector according to the invention a medical amateur can easily mix and (self)apply a medicament that consists of two or more elements as the chambers of the autoinjector are prefilled with the right dosage of elements. Furthermore, by providing a security mechanism, the autoinjector according to the invention is safely secured against premature mixing and dispensing such that the storage time of the elements contained in the autoinjector can be enhanced as they cannot come in contact with one another before a mixing mechanism is triggered. Additionally, said security mechanism can be configured to provide a sterile environment for all parts of the autoinjector that are involved in the injection process of the mixed medicament.

According to a further aspect of the invention, which may in particular be combined with the first aspect of the invention, an autoinjector is provided comprising a dual chamber cartridge comprising two elements of a medicament that are each stored in a separate chamber of the dual chamber cartridge and the dual chamber cartridge comprising a bypass passage. The dual chamber cartridge further comprises a cap and seal assembly covering and fixed to an outlet from the dual chamber cartridge.

That is, according to this further aspect of the invention the autoinjector comprises a dual chamber cartridge comprising two chambers configured to store two elements of a medicament, i.e. one medicament element in each chamber. Furthermore, the autoinjector comprises a bypass passage that is configured to enable the two elements to be mixed with one another when a mixing mechanism is triggered by for example allowing one element to flow from its storage chamber into the other chamber.

Furthermore, the autoinjector according to the invention comprises a cap and seal assembly covering and fixed to an outlet from the dual chamber cartridge. Said cap and seal assembly is therefore configured to close an outlet of the cartridge such that the elements stored therein can be held in a sterile environment. Furthermore, the cap and seal assembly can be configured to prevent a premature mixing and dispensing of said elements.

In this connection it may be noted that the cap and seal assembly may comprise a cap and one or more seals surrounded by the cap. Hence, according to this embodiment the cap and seal assembly can be a two-step assembly that ensures safe sealing of the dual chamber cartridge at least as long as it is in its storage state.

According to another embodiment of the invention the dual chamber cartridge has an opening which is covered by the cap and sealed off by one of said one or more seals of the cap and seal assembly. That is, the one or more seals can for example be configured to directly close the opening of the dual chamber cartridge while the cap covering said one or more seals may be configured to not only cover the outlet of the dual chamber cartridge but also said seal. The cap may in particular further be configured to (completely) surround the opening as well as the one or more seals.

According to a further embodiment the cap and seal assembly comprises a first flange arrangement and the dual chamber cartridge comprises a second flange arrangement shaped complementary to the first flange arrangement and being engageable by the first flange arrangement. Hence, said first and second flange arrangements may enable a secure engagement between the cap and seal assembly and the dual chamber cartridge.

In this connection it should be noted that the first flange arrangement can surround said second flange arrangement. It may in particular be possible that said first flange arrangement completely surrounds said second flange arrangement such that the first flange arrangement can generally be put on said second flange arrangement to provide a safe connection between the two.

It may further be possible that the first flange arrangement is snap-fit into place at the second flange arrangement. Such a snap fit connection can ensure a safe connection between the first and the second flange arrangement. It may in particular even provide a releasable connection which can potentially be useful in case the autoinjector should be reusable or in case the different parts of the autoinjector are supposed to be discarded separately from one another.

According to another embodiment of the invention the autoinjector further comprises a cannula held in place at the dual chamber cartridge by the cap and seal assembly. Said cannula may for example be configured as an injection needle. That is, said cannula may extend from an outside of the dual chamber cartridge to an inside of the dual chamber cartridge by extending beyond said cap and/or said one or more seals of the cap and seal assembly.

In this connection it is noted that the cannula may be held in place at the dual chamber cartridge by one of said one or more seals of the cap and seal assembly. That is, said one or more seals can further be configured to hold said cannula in place.

It may further be possible that the cap and seal assembly further comprises a cannula shield covering the cap and seal assembly. Such a cannula shield can ensure safe handling of the autoinjector as it prevents a user from being unintentionally stuck by said cannula. Additionally, said cannula shield can ensure a sterile environment for the outlet of the autoinjector, respectively said cannula, before using it on a patient as the outlet respectively the cannula is protected from the environment.

According to a further embodiment of the invention the dual chamber cartridge further comprises a proximal and a distal stopper. Said stopper may for example be configured to seal the two chambers of the dual chamber cartridge at at least one end of each chamber. Additionally or alternatively they may be configured to push the elements stored in said respective chambers towards the outlet of the autoinjector upon mixing and/or dispensing of the elements.

According to another embodiment the dual chamber cartridge comprises a first chamber more proximally arranged than a second chamber, wherein the first chamber is filled with a solid material and the second chamber is filled with a liquid material. That is, each element of the medicament can be stored in a separate chamber until they are intentionally mixed with one another in order to enhance the storage time of said elements.

In this connection it should be noted that the first chamber may be separated from the second chamber by the proximal stopper. According to this embodiment no further separation or chamber walls are necessary to separate one chamber from the other as the proximal stopper can act as such a separation wall.

It may further be possible that the proximal stopper is arranged distally of the bypass passage in a storage state of the autoinjector. This prevents the element stored in the second chamber from reaching the first chamber as the bypass passage is blocked by the proximal stopper.

In this connection it should further be noted that the proximal stopper can be arranged proximally of the bypass passage after a dispensing operation has taken place. That is, the proximal stopper may be arranged moveably in the autoinjector such that it may first be arranged distally of the bypass passage in a storage state and then proximally of the bypass passage after a dispensing operation has taken place.

By having the proximal stopper arranged proximally of the bypass passage after a dispensing operation has taken place it can further be prevented that any residuals of the medicament left in the first chamber can flow back into the second chamber behind the proximal stopper. This can be particularly important in applications where not all of the mixed medicament is injected at once but rather by two or more injections throughout a predetermined period of time.

According to a further embodiment the autoinjector further comprises a piston rod configured to act on said distal stopper and said proximal stopper and to thereby move the proximal stopper such that it is arranged at the bypass passage for urging the material into the proximal chamber past the proximal stopper via the bypass passage during a mixing operation.

That is, according to this embodiment both the distal and the proximal stopper are arranged moveably in the dual chamber cartridge to be able to urge the elements that are arranged right in front of the respective stoppers towards the outlet of the dual chamber cartridge.

In this connection it should be noted that as the element that is stored in the distal chamber is urged through the bypass passage into the proximal chamber during the mixing operation, it may be possible for the distal and the proximal stopper to come into contact to later move together as one.

It may thus be possible that the piston rod can be moved further for dispensing the medicament via the cap and seal assembly. That is, the piston rod can be moved such that it brings both stoppers towards the cap and seal assembly to urge the mixed material towards the outlet.

According to a further embodiment the one of said one or more seals of the cap and seal assembly sealing off the opening covers said opening. By sealing the opening it can for example be ensured that the mixed medicament can only be dispensed via the cap and seal assembly and/or a cannula.

According to yet another embodiment the autoinjector further comprises a cannula shield such as a rigid cannula shield covering a cannula of the dual chamber cartridge. Such a cannula shield can for example be provided to ensure safe handling of the autoinjector and to prevent injuries by shielding the user from the cannula. Furthermore, said cannula shield can be configured to provide a sterile environment for the cannula before the first use of the autoinjector.

According to a further aspect of the invention a method of mixing a medicament in an autoinjector is provided with the autoinjector comprising a dual chamber cartridge comprising first and second elements of a medicament, with the first and second elements each being stored in a separate chamber of the dual chamber cartridge, the dual chamber cartridge comprising a cap and seal assembly as well as a piston rod. The method according to the invention comprises the steps of: - moving the piston rod proximally towards a distal stopper and thereby moving said distal stopper towards a proximal stopper and reducing the distance between the distal stopper and said proximal stopper to thereby urge a first element of the medicament from a first chamber into a bypass passage to bypass the proximal stopper such that the first element comes into contact with the second element in the first chamber arranged between the cap and seal assembly and the proximal stopper.

That is, according to the invention it is possible to store two elements of a medicament separately in two different chambers in order to enhance the storage time of one or more of said elements.

Then, by moving the piston rod proximally towards the distal stopper such that said distal stopper is also guided towards the proximal stopper, the element that is stored in the distal chamber is urged towards said proximal stopper. When the pressure that acts on said proximal stopper is high enough, also the proximal stopper will start moving in a proximal direction thereby releasing the bypass channel such that the second element stored in the distal, i.e. the second, chamber can flow through said bypass channel to bypass the proximal stopper and to reach the proximal, i.e. the first, chamber where the first element is stored. This allows a mixing of the two elements.

It may additionally also be possible to move the piston rod further after the mixing of the elements is complete in order to urge the mixed medicament out of an outlet of the autoinjector in order to inject said medicament into a patient.

According to a further aspect of the invention an autoinjector comprising a dual chamber cartridge comprising two elements of a medicament that are each stored in a separate chamber of the dual chamber cartridge is provided. The dual chamber cartridge according to the invention comprises a bypass passage and a rubber septum and a cannula shield. The autoinjector further comprises a cannula carriage having a cannula with the cannula carriage being in contact with the dual chamber cartridge in a storage state of the autoinjector and the dual chamber cartridge being moveable towards the cannula carriage on activation of the autoinjector in order to penetrate the septum with the cannula.

That is, according to this further aspect of the invention the autoinjector comprises a dual chamber cartridge comprising two chambers configured to store two elements of a medicament, i.e. one element of medicament in each chamber.

Furthermore, the autoinjector comprises a bypass passage that may be configured to enable the two elements to be mixed with one another when a mixing mechanism and/or an activation mechanism is triggered.

According to this aspect of the invention the autoinjector further comprises a cannula carriage having a cannula with the cannula carriage being in contact with the dual chamber cartridge in a storage state. The cannula carriage may thus enable a safe connection of the cannula at the dual chamber cartridge.

However, in a storage state, said cannula carriage is only in contact with the dual chamber cartridge but the cannula itself does not yet extend into said dual chamber cartridge. This is only the case when the autoinjector is activated by moving the dual chamber cartridge towards the cannula carriage (or the other way around) such that the cannula can penetrate the septum of the dual chamber cartridge to extend beyond said septum into said dual chamber cartridge thereby providing a flow channel for the elements stored inside the autoinjector.

According to an embodiment of the invention a distal end of the cannula carriage is sealed off by a seal to make sure that the elements stored inside the dual chamber cartridge can only flow through said cannula.

In this connection it should be noted that said seal may be moveably arranged in a distal carriage reception space relative to the cannula. By arranging said seal moveable in said distal carriage reception space, the seal can move relative to the cannula upon activation of the autoinjector when the dual chamber cartridge is moved towards the cannula carriage. It may thus be possible that said seal moves from a distal end of the cannula carriage reception space towards a proximal end of said cannula carriage reception space upon activation of the autoinjector.

According to a further embodiment the cannula carriage comprises one or more detents at a distal end thereof. Said detents may be configured to hold the dual chamber cartridge, i.e. to secure the dual chamber cartridge at the cannula carriage.

It may further be possible that the cannula carriage is in contact with the dual chamber cartridge via the one or more detents in the storage state of the autoinjector. Said detents may for example be configured to secure the cannula carriage at the dual chamber cartridge. Additionally or alternatively, they may be configured to hold the cannula carriage at a predetermined distance with respect to the dual chamber cartridge.

Furthermore, on activation of the autoinjector, the dual chamber cartridge may be configured to overcome said one or more detents such that the cannula pierces the septum. That is, the one or more detents may be configured to provide a securing mechanism that has to be overcome upon activation of the autoinjector.

It could further be possible that a movement of the dual chamber cartridge towards the cannula carriage moves said seal towards the cannula to pierce said seal on activation of the autoinjector.

In this connection it should also be noted that the movement of the dual chamber cartridge towards the cannula carriage may move said seal towards the cannula to first pierce said seal and then secondly pierce said septum to allow the cannula to extend beyond said seal and said septum into the dual chamber cartridge.

According to another embodiment of the invention the autoinjector further comprises a distal stopper arranged in said dual chamber cartridge, and a piston rod, wherein, on activation of said autoinjector, a movement of said piston rod towards the distal stopper brings about a movement of said dual chamber cartridge towards said cannula carriage. That is, to activate the autoinjector a user may move the piston rod towards said distal stopper which then translates into a movement of the dual chamber cartridge towards the cannula carriage.

It may further be possible that once said septum is pierced, a movement of said distal stopper within the dual chamber cartridge is facilitated. Such a movement of the distal stopper may for example urge the elements stored in the dual chamber cartridge towards the cannula in order to be dispensed, i.e. injected into a patient.

According to another embodiment of the invention the dual chamber cartridge comprises a proximal and a distal stopper and a bypass passage. Said proximal and distal stoppers may have various tasks. For example, the distal stopper can be configured to be moved towards the proximal stopper upon activation of the autoinjector.

Furthermore, it may possible that the proximal stopper is configured to urge the elements that are stored in the autoinjector towards the cannula upon a dispensing motion of the autoinjector, in particular together with the distal stopper.

The proximal and the distal stopper may be arranged at a distance from one another in a storage state in order to provide two chambers in which the elements of the medicament can be stored separately from one another.

The bypass passage may be arranged at or near the proximal stopper such that upon activation of the autoinjector said proximal stopper may move towards said bypass passage such that the element stored behind, i.e. distally relative to the proximal stopper can flow through said bypass passage to bypass the proximal stopper and to reach the element that is stored in front of, i.e. proximally relative to the proximal stopper in order to be mixed with one another.

It may further be possible that the dual chamber cartridge comprises a first chamber more proximally arranged than a second chamber, wherein the first chamber is filled with a solid material of said medicament and the second chamber is filled with a liquid material of said medicament.

According to one embodiment the first chamber is separated from the second chamber by the proximal stopper. That is, in this case no additional separation walls or the like are necessary to separate the first chamber from the second chamber.

In this connection it should be noted that in some cases the autoinjector further comprises a piston rod configured to act on said distal stopper and said proximal stopper and to thereby move the proximal stopper such that it is arranged at the bypass passage for urging the liquid material into the first chamber past the proximal stopper via the bypass passage such that the liquid material can be mixed with the solid material.

According to a further aspect of the invention a method of activating an autoinjector is provided, the autoinjector comprising a dual chamber cartridge comprising first and second elements of a medicament, with the first and second elements each being stored in a separate chamber of the dual chamber cartridge and a cannula carriage having a cannula and a piston rod. The method according to the invention comprises the steps of:
- moving the piston rod proximally towards a distal stopper arranged in the dual chamber cartridge and thereby moving said dual chamber cartridge towards the cannula carriage to pierce a septum of said dual chamber cartridge with said cannula.

In some cases the method may further comprise the step of allowing air to be expelled from said dual chamber cartridge via said cannula and then further moving the distal stopper towards a proximal stopper to thereby urge a second element of the medicament from a second chamber into a bypass passage to bypass the proximal stopper such that the second element comes into contact with the first element in the first chamber arranged between the cannula carriage and the proximal stopper.

According to a further aspect of the invention an autoinjector comprising a dual chamber cartridge comprising two elements of a medicament that are each stored in a separate chamber of the dual chamber cartridge is provided. The dual chamber cartridge further comprises a bypass passage and an outer cap, an inner cap and a cannula shield covering a cannula of the autoinjector, wherein, on activation of the autoinjector said inner cap is removable from the autoinjector before said outer cap is removed from said autoinjector.

That is, according to this further aspect of the invention the autoinjector comprises a dual chamber cartridge comprising two chambers configured to store two elements of a medicament, i.e. one medicament in each chamber. Furthermore, the autoinjector comprises a bypass passage that may be configured to enable the two elements to be mixed with one another when a mixing mechanism is triggered.

The autoinjector according to the invention further comprises an outer cap, an inner cap and a cannula shield covering a cannula of the autoinjector. On activation of the autoinjector said inner cap is removable from the autoinjector before said outer cap is removed from said autoinjector.

Said inner and outer caps may have the same or different functions. For example they may both be configured to shield a user from the cannula when the autoinjector is in the storage state. Additionally or alternatively either one of the inner and the outer cap or both may be configured to keep the autoinjector in a storage state to prevent premature mixing and/or dispensing of the elements stored in the dual chamber cartridge.

It can for example be possible that the inner cap is configured to prevent premature mixing of the elements stored in the dual chamber cartridge while the outer cap is configured to prevent premature dispensing of the elements.

The cannula shield can further be configured to shield the cannula and/or to ensure a sterile environment for the cannula in the storage stage of the autoinjector.

Additionally, the cannula shield can also be configured to keep the autoinjector from prematurely mixing and dispensing the elements stored therein, for instance. In this connection it should be noted that it would also be possible to complete mixing and to accept a certain degree of over pressure in the proximal chamber of the syringe.

According to an embodiment of the invention the autoinjector is configured such that removal of the inner cap simultaneously brings about a removal of the cannula shield such that only one action is required from a user to remove both the inner cap and the cannula shield to start an activation of the autoinjector. In this connection it should be noted that the mixing process of the two materials should be avoided prior to removal of the inner cap, hence the reconstitution or mixing process is preferably hindered prior to removal of the inner cap, so that removal of the inner cap is considered a first stage of activation of the autoinjector.

In this connection it should be noted that the inner cap may comprise a grip projecting from the autoinjector to facilitate the removal of the inner cap for the user.

The grip may comprise a ring which can be easily gripped by a user.

According to another embodiment removal of the inner cap permits a movement of air out of the dual chamber cartridge. This may facilitate the mixing of the two elements stored in the dual chamber cartridge.

According to another embodiment of the invention the inner cap comprises a cannula shield holder at an end of the inner cap disposed opposite to a base of the inner cap such that the inner cap may be configured to additionally hold a cannula shield.

According to a further embodiment the inner cap comprises inwardly facing projections that engage the cannula shield. This may for example be advantageous in case the cannula shield is supposed to be removed together with the inner cap as said projections can safely secure the inner cap to the cannula shield.

Said inwardly facing projections may for example be integrally formed with the cannula shield holder of the inner cap. This can lead to lower production costs and time as less single components are needed to form the inner cap, the inwardly facing projections and the cannula shield holder. Thus, one could even think of providing an inner cap, cannula shield holder and inwardly facing projections that are made as one-piece unit. Alternatively, only the cannula shield holder and the projections could be made as a single unit, for instance.

In this connection it should further be noted that the inwardly facing projections may also be formed by a metal insert received within the cannula shield holder of the inner cap which may have benefits in view of durability and strength of the inwardly facing projections.

According to a further embodiment the outer cap is configured to receive a part of a cannula shield of the autoinjector.

According to yet another embodiment an axial movement of a cannula guard of the autoinjector in the direction of the dual chamber cartridge brings about a release of a drive mechanism of a plunger of the dual chamber cartridge for dispensing a medicament stored in the dual chamber cartridge.

The invention is further described by the following embodiments and Figures which show:
- Fig. 1:: various sectional views of different stages of use of a dual chamber autoinjector;
- Fig. 2: a sectional view of a pre-filled syringe including a cannula shield;
- Fig. 3:: a sectional view of the pre-filled syringe of Fig. 2 without the cannula shield;
- Fig. 4:: a sectional view of a dual chamber cartridge comprising a cannula carriage and a cannula shield;
- Fig. 5:: a sectional view of the dual chamber cartridge of Fig. 4 without the cannula shield;
- Fig. 6:: a close-up view of the cannula carriage of Figs. 4 and 5; and
- Fig. 7:: detailed views of the outer and inner caps of the autoinjector.

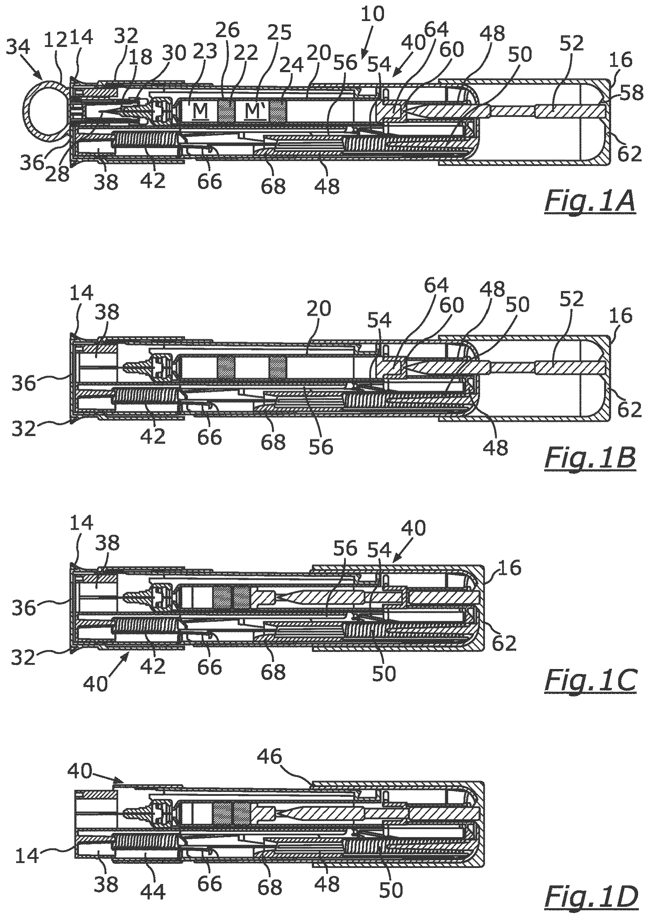

Figs. 1a to g show various sectional views of different stages of use of a dual chamber autoinjector 10. Fig. 1a shows the as delivered state of the autoinjector 10, with a handle 12 protruding from a proximal end 14 of the autoinjector. The proximal end 14 is oppositely disposed to the distal end 16.

In this connection it should be noted that the terms proximal and distal refer to the position of a cannula 18 of the autoinjector 10 relative to a patient (not shown) with proximal meaning closest to a main mass of the body of a patient and distal meaning it is more distant from the main mass of the body of a patient.

Hence, e.g. the proximal end 14 of the autoinjector 10 is that end that is placed at a patients skin with the distal end 16 being remote from the patient's skin in use, i.e. the end of the device having a needle that pierces a patient's skin is the proximal end 14.

The cannula 18 is arranged at a pre-filled syringe 20. The pre-filled syringe is a so-called dual chamber pre-filled syringe 20 which in a storage state comprises two chambers 23, 25 separated from one another for storage of two kinds of material M, M' by a proximal stopper 22, with the second chamber 25 also being bound by a distal stopper 24.

Such a pre-filled syringe and hence the autoinjector 10 can be used for mixing, e.g. liquid/liquid compositions, respectively reconstitution processes, e.g. powder/liquid compositions, in which the two different materials M, M' are stored separate from one another but are mixed in a mixing or a reconstitution process to form a medicament before dispensing of the medicament. The reason for this is that the medicament may not have a long storage life once mixed but its components do.

The two chambers can be connected one to another via a bypass passage 26 once the distal stopper and the proximal stopper are moved proximally towards the cannula 18.

The cannula 18 is covered by a cannula shield 28 that can be removed from the pre-filled syringe 20 on removal of the handle 12 from a cap 32 present at the proximal end 14 of the autoinjector 10.

The handle 12 comprises an inner cap 34 attached thereto, with the inner cap 34 being arranged within the cap 32. The inner cap 34 is configured to hold and move a metal pressing 30 arranged to hold and remove the cannula shield 28 on removal of the handle 12 from the cap 32.

For this purpose the cap 32 comprises an aperture 36 via which the inner cap 34, the metal pressing 30 and the cannula shield 28 can be removed from the autoinjector 10 and the pre-filled syringe 20 via the aperture 36.

The autoinjector 10 further comprises a cannula guard 38 arranged at the proximal end 14. The cannula guard 38 is configured to be moved relative to the cannula 18 once the cap 32 is removed during an activation operation and a lock out operation as indicated in Figs. 1c to g.

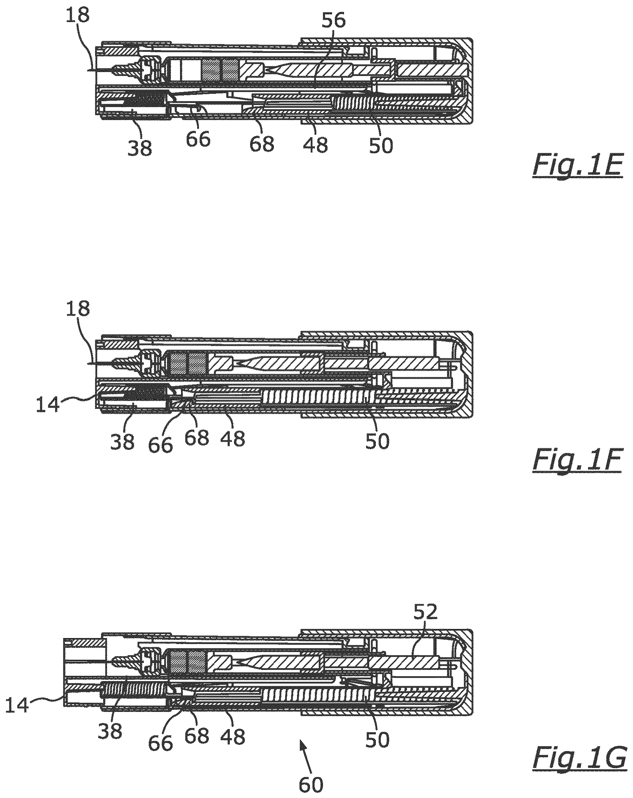

The cannula guard 38 is arranged to move relative to a housing 40 of the autoinjector 10. A lock-out spring 42 is arranged between the cannula guard 38 and the housing 40. In the state shown in Figs. 1a to d and g, the lock out 42 spring is its extended state whereas in the state indicated in Fig. 1e and f, the lock-out spring 42 is in the compressed state.

The housing 40 is formed by an inner body 44 and an outer body 46, with the pre-filled syringe being held at the inner body 44 which in turn is snap fit into place at the outer body 46 in such a manner that the pre-filled syringe cannot move axially with respect to either of the inner body 44 and the outer body 46.

The autoinjector 10 further comprises a drive chassis 48 arranged movable in the housing 40 between a storage position (Fig. 1a) respectively a pre-dispense position (Figs. 1b to e) and a dispensed position (see Figs. 1f and g).

A drive spring 50 is biased between the outer body 46 and the drive chassis 48 and is configured to release energy in order to drive the drive chassis from the pre-dispense position to the dispensed position once the drive chassis 48 has been released for movement.

The drive chassis 48 is released for movement relative to the housing 40 once the cannula guard 38 is moved distally (see e.g. Figs. 1e and f) and a cannula guard plunger 56 engages trigger arms 54 of the drive chassis 48 and releases an engagement between the trigger arms 54 and the housing 40.

The spring chassis 48 then entrains a piston rod 52 in order to dispense a medicament composed of the mixed two kinds of material M, M'.

The autoinjector 10 further comprises a sleeve 62 within which the housing 40 is received and which is moveable relative to the housing 40. The sleeve 62 comprises a piston rod holder 58 for releasably holding the piston rod 52.

A front end 64 of the piston rod 52 is held at a journal 60 present at the drive chassis 48. The piston rod 52 is entrained by the journal 60 on carrying out the dispensing action.

The inner body 44 comprises an end of dose click arm 66 that is configured to engage the drive chassis 48 towards an end of dose following the carrying out of the dispensing action. The end of dose click arm 66 cooperates with an end of dose click ramp 68 provided at the drive chassis 48. The end of dose click arm 66 can only engage the drive chassis 48 once the drive chassis 48 has moved proximally.

Fig. 1b shows a state of the autoinjector 10 in which the inner cap 34 and the cannula shield 28 are removed prior to a reconstitution process having been carried out.

Fig. 1c shows a state in which the sleeve 62 is moved relative to the housing 40 in order to move the distal plunger 24 and the second medicament M' proximally such that the second medicament M' can come into contact with the first medicament via the bypass passage 26 to carry out the reconstitution process.

Fi. 1d shows the state in which the cap 32 of the autoinjector 10 has been removed. Only after removal of the cap 32 is it possible to actuate the cannula guard which can be used to activate the autoinjector 10 for the dispensing process.

This occurs as indicated in Fig. 1e when the cannula guard 38 is fully depressed and the cannula guard plunger 56 engages the trigger arms 54 to release the drive chassis 48.

As indicated in Fig. 1f, the cannula guard 38 is still fully depressed and the dispense is completed, i.e. the drive chassis 48 has moved as far proximally as possible.

During this proximal movement of the drive chassis 48, the end of dose click arm 66 engages the end of dose click ramp 68 and emits an audible clicking noise indicating a near completion respectively a completion of the dispensing process.

Once a patient hears the end of dose click, he knows that dispensing is completed and can remove the autoinjector 10 from the point of administration at the patient or start a count to do so, the lock-out spring 42 then moves the cannula guard 38 into a locked position in which the cannula 18 is covered by the cannula guard 38. It is preferred if the cannula guard 38 cannot be moved relative to the cannula 18 when this is in the locked position.

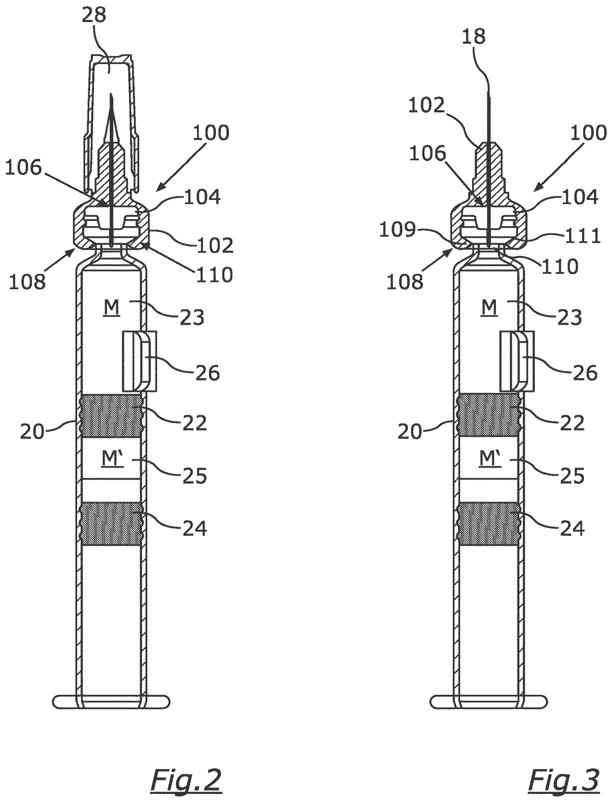

Figs. 2 and 3 show sectional views of a prefilled syringe 20 according to the invention. The syringe 20 of Fig. 2 additionally comprises the removable cannula shield 28 while the syringe 20 shown in Fig. 3 is without said cannula shield 28.

It can be clearly seen that the prefilled syringe 20 comprises a dual chamber cartridge 11 having two chambers 23, 25 wherein the first chamber 23 is arranged proximally relative to the second chamber 25. Each of said two chambers 23, 25 comprises one element M respectively M' of a medicament such that said two elements M, M' are stored separately from one another to enhance the storage time.

It can furthermore be clearly seen that the first and second chambers 23, 25 are separated from one another by the proximal stopper 22 and that the second chamber 25 additionally comprises a distal stopper 24 at its distal end.

The prefilled syringe 20 of Figs. 2 and 3 is shown in the storage state as the proximal stopper 22 is arranged behind, i.e. distally relative to the bypass passage 26 such that the material M' stored in the second chamber 25 cannot yet flow into the first chamber 23.

Additionally, the prefilled syringe 20 of Figs. 2 and 3 comprises a cap and seal assembly 100 having a cap 102 and one or more seals 104 with said cap and seal assembly 100 being fixed to and covering an opening 106 of the dual chamber cartridge 11.

It can be seen that the cap 102 completely surrounds seal 104 as well as the opening 106.

The cannula 18 extends through and beyond the cap and seal assembly 100 as it is depicted in Fig. 2. This way, the elements M, M' stored inside the dual chamber cartridge 11 can only flow through the cannula 18 during the act of dispensing as seal 104 completely seals and covers the potential open residue of the opening 106.

Said cannula 18 can furthermore be stabilized by cap 102 and/or by the one or more seals 104.

The cap and seal assembly 100 of Figs. 2 and 3 further comprises a first flange arrangement 108 while the dual chamber cartridge 11 comprises a second flange arrangement 110 that is shaped complementary to said first flange arrangement. The two flange arrangements 108,110 are engageable with one another such that the cap and seal assembly 100 can safely be secured to the dual chamber cartridge 11.

In the embodiment of Figs. 2 and 3 this is done by providing a first flange arrangement 108 with an undercut 109 and a second flange arrangement 110 with a web 111 such that said undercut 109 can engage with said web 111. In this case it is also possible that the first flange arrangement, i.e. the undercut 109, completely surrounds the second flange arrangement 110, i.e. the web 111.

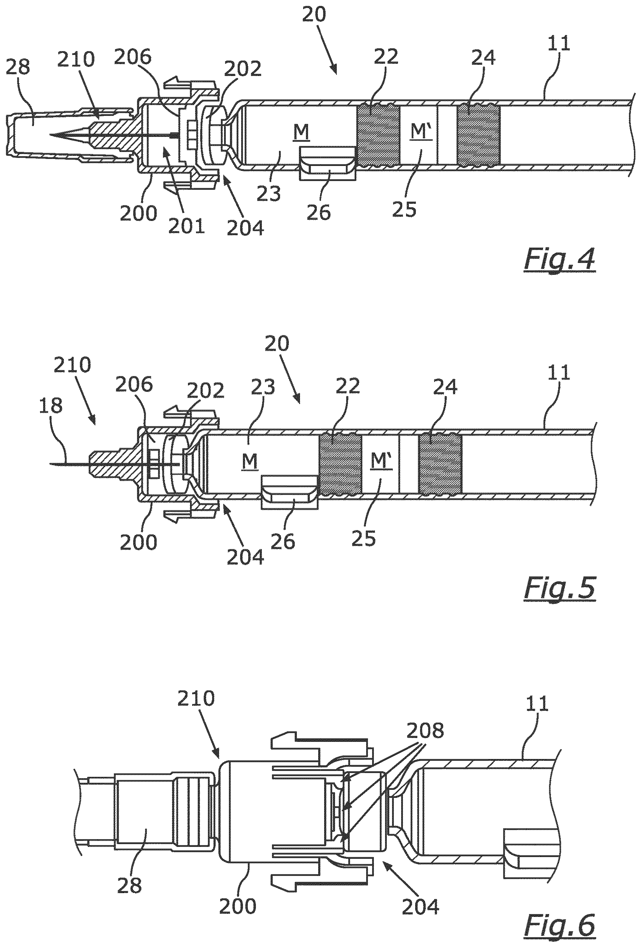

The second aspect of the invention is shown in Figs. 4 to 6 with Figs. 4 and 5 showing detailed sectional views of a prefilled syringe 20 according to this second aspect of the invention and Fig. 6 showing a detailed view of a cannula carriage 200 arranged at said prefilled syringe 20, in particular at the dual chamber cartridge 11.

In this connection it is noted that for the general features of the prefilled syringe 20 of Figs. 4 to 6 it is referred to the explanations made in connection with Figs. 1 to 4 as said general features are the same. In the following only the features that differ from the embodiments of Figs. 1 to 4 are described.

The prefilled syringe 20 according to Figs. 4 to 6 comprises a cannula carriage 200 instead of the cap and seal assembly 100 of Figs. 2 and 3. Said cannula carriage 200 is arranged at the outlet 106 of the dual chamber cartridge 11.

The cannula 18 is arranged inside of the cannula carriage such that it extends beyond said carriage 200 at a proximal end in the storage state of the autoinjector 10. At a distal end of the cannula carriage 200 said carriage 200 comprises a cannula carriage reception space 201 through which the cannula 18 extends. The cannula 18 is further arranged moveably with respect to said reception space 201 such that upon activation of the autoinjector 10 the dual chamber cartridge 11 is moved towards the cannula carriage 200.

Upon said activation movement the cannula 18 pierces a rubber septum 202 that is arranged at and seals the outlet 106 of the dual chamber cartridge 11 in the storage state. Hence, in the activated state of the autoinjector 10 the cannula carriage 200 is arranged closer to the dual chamber cartridge 11 such that the cannula 18 extends beyond the rubber septum 202 and thus the opening 106 of the dual chamber cartridge 11 into the first chamber 23.

At the distal end 204 of the carriage reception space 201 a seal 206 is arranged moveably with respect to said reception space 201 such that upon the activation process of the autoinjector 10 the seal 206 moves in a proximal direction towards a proximal end of the carriage reception space 201.

The seal 206 is arranged at the carriage reception space 201 to seal the area around the cannula 18. Furthermore, it may be configured to be pierced by the cannula 18 upon activation of the autoinjector 10 such that said seal 206 is pierced before the rubber septum 202 is pierced.

The cannula carriage 200 of Figs. 4 to 6 additionally comprises detents 208 at the distal end 204 thereof through which the cannula carriage 200 is in contact with the dual chamber cartridge 11 in the storage state of the autoinjector 11 (see in particular Fig. 6).

In the activated state of the autoinjector 10 the detents 208 can be overcome such that the cannula carriage 200 moves closer to the dual chamber cartridge 11 (see Fig. 5) to allow the piercing of the septum 202.

Also the prefilled syringe 20 according to Figs. 4 to 6 can comprise an additional cannula shield 28 that is arranged at the proximal end 210 of the cannula carriage 200 in the storage state of the autoinjector 10 (see Fig. 4). Upon activation of the autoinjector 10 the cannula shield 28 can be removed as it is already explained in connection with Fig. 1.

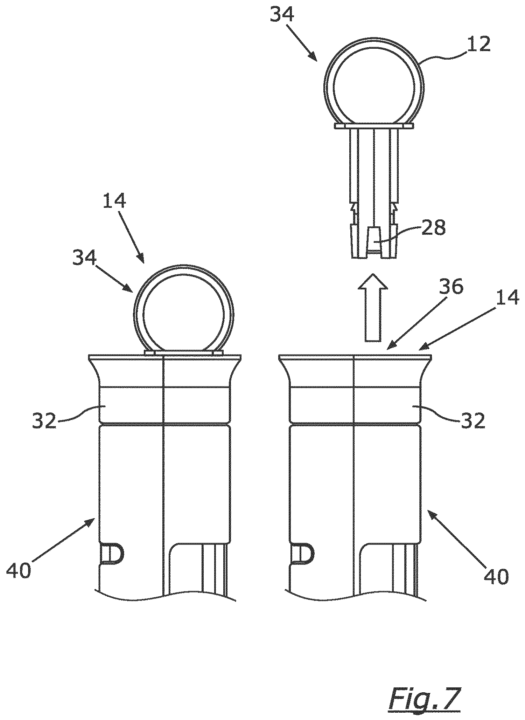

Another aspect of the invention is shown in Fig. 7 which shows a detailed view of the proximal end 14 of the autoinjector 10 comprising an inner and an outer cap 34, 32. The functioning of said inner and outer caps 34, 32 is already described in connection with Fig. 1.

In this connection it is noted that it may additionally be possible that the autoinjector according to Fig. 7, and thus also Fig. 1, additionally comprises the cannula carriage 200 as described in connection with Figs. 4 to 6 and/or the cap and seal assembly 100 as described in connection Figs. 2 and 3.

The invention is further defined in the following embodiments.

An autoinjector comprising a dual chamber cartridge (11) comprising two elements of a medicament (M, M') that are each stored in a separate chamber (23, 24) of the dual chamber cartridge (11) and the dual chamber cartridge (11) comprising a bypass passage (26) and the autoinjector (10) further comprising at least one security mechanism configured to secure the autoinjector against premature mixing of the elements and dispensing of said mixed elements (M, M').

An autoinjector, in particular according to claim 1, comprising a dual chamber cartridge (11) comprising two elements of a medicament (M, M') that are each stored in a separate chamber (23, 24) of the dual chamber cartridge (11) and the dual chamber cartridge (11) comprising a bypass passage (26), the dual chamber cartridge (11) comprising a syringe cap and seal assembly (100) covering and fixed to an outlet from the dual chamber cartridge (11).

The autoinjector of claim 2, wherein the cap and seal assembly (100) comprises a cap (102) and one or more seals (104) surrounded by the cap (102).

The autoinjector of claim 3, wherein the dual chamber cartridge (11) has an opening (106) which is covered by the cap (102) and sealed off by one of said one or more seals (104) of the cap and seal assembly (100).

The autoinjector of one of claims 2 to 4, wherein the cap and seal assembly (100) comprises a first flange arrangement (108) and the dual chamber cartridge (11) comprises a second flange arrangement (110) shaped complementary to the first flange arrangement (108) and being engageable by the first flange arrangement (108).

The autoinjector of claim 5, wherein the first flange arrangement (108) surrounds said second flange arrangement (110).

The autoinjector of claim 5 or claim 6, wherein the first flange arrangement (108) is snap-fit into place at the second flange arrangement (110).

The autoinjector of one of claims 2 to 7, further comprising a cannula (18) held in place at the dual chamber cartridge (11) by the cap and seal assembly (100).

The autoinjector of claim 8 and one of claims 3 to 7, wherein the cannula (18) is held in place at the dual chamber cartridge (11) by one of said one or more seals (104) of the cap and seal assembly (100).

The autoinjector of one of claims 2 to 9, wherein the cap and seal assembly (100) further comprises a cannula shield (28) covering the cap and seal assembly (100).

The autoinjector of one of claims 2 to 10, wherein the dual chamber cartridge (11) further comprises a proximal (22) and a distal stopper (24).

The autoinjector of one of claims 2 to 11, wherein the dual chamber cartridge (11) comprises a first chamber (23) more proximally arranged than a second chamber (25), wherein the first chamber (23) is filled with a solid material (M) and the second chamber (25) is filled with a liquid material (M').

The autoinjector of claims 11 and claim 12, wherein the first chamber (23) is separated from the second chamber (25) by the proximal stopper (22).

The autoinjector of one of claims 11 to 13, wherein the proximal stopper (22) is arranged distally of the bypass passage (26) in a storage state of the autoinjector (10).

The autoinjector of one of claims 11 to 14, wherein the proximal stopper (22) is arranged proximally of the bypass passage (26) after a dispensing operation has taken place.

The autoinjector of one of claims 2 to 15, further comprising a piston rod (52) configured to act on said distal stopper (24) and said proximal stopper (22) and to thereby move the proximal stopper (22) such that it is arranged at the bypass passage (26) for urging the material into the proximal chamber (23) past the proximal stopper (22) via the bypass passage (26) during a mixing operation.

The autoinjector of claim 16, wherein the piston rod (52) can be moved further for dispensing the medicament via the cap and seal assembly (100).

The autoinjector of one of claims 4 to 17, wherein the one of said one or more seals (104) of the cap and seal assembly (100) sealing off the opening (106) covers said opening (106).

The autoinjector of one of claims 2 to 18, further comprising a cannula shield (28) such as a rigid cannula shield (28) covering a cannula (18) of the pre-filled syringe.

A method of mixing a medicament in an autoinjector (10), the autoinjector (10) comprising a dual chamber cartridge (11) comprising first and second elements (M, M') of a medicament, with the first and second elements (M, M') each being stored in a separate chamber (23, 25) of the dual chamber cartridge (11), the dual chamber cartridge (11) comprising a cap and seal assembly (100) as well as a piston rod (52), the method comprising the steps of:
- moving the piston rod (52) proximally towards a distal stopper (24) and thereby moving said distal stopper (24) towards a proximal stopper (22) and reducing the distance between the distal stopper (24) and said proximal stopper (22) to thereby urge a first element (M) of the medicament from a first chamber (23) into a bypass passage (26) to bypass the proximal stopper (25) such that the first element (M) comes into contact with the second element (M') in the first chamber (25) arranged between the cap and seal assembly (100) and the proximal stopper (22).

An autoinjector, in particular according to claim 1, comprising a dual chamber cartridge (11) comprising two elements (M, M') of a medicament that are each stored in a separate chamber (23, 25) of the dual chamber cartridge (11), the dual chamber cartridge (11) comprising a bypass passage (26) and a rubber septum (202) and a cannula shield (28), the autoinjector (10) further comprising a cannula carriage (200) having a cannula (18) with the cannula carriage (200) being in contact with the dual chamber cartridge (11) in a storage state of the autoinjector (10) and the dual chamber cartridge (11) being moveable towards the cannula carriage (200) on activation of the autoinjector (10) in order to penetrate the septum (202) with the cannula (18).

The autoinjector of claim 21, wherein a distal end (204) of the cannula carriage (200) is sealed off by a seal (206).

The autoinjector of claim 22, wherein the seal (206) is moveably arranged in a distal cannula carriage reception space (201) relative to the cannula (18).

The autoinjector of one of claims 21 to 23, wherein the cannula carriage (200) comprises one or more detents (208) at a distal end (204) thereof.

The autoinjector of claim 24, wherein the cannula carriage (200) is in contact with the dual chamber cartridge (11) via the one or more detents (208) in the storage state of the autoinjector (10).

The autoinjector of claim 24 or claim 25, wherein, on activation of the autoinjector (10), the dual chamber cartridge (11) is configured to overcome said one or more detents (208) such that the cannula (18) pierces the septum (202).

The autoinjector of one of claims 22 to 26, wherein a movement of the dual chamber cartridge (11) towards the cannula carriage (200) moves said seal (206) towards the cannula (18) to pierce said seal (206) on activation of the autoinjector (10).

The autoinjector of claim 27 and one of claims 22 to 26, wherein the movement of the dual chamber cartridge (11) towards the cannula carriage (200) moves said seal (206) towards the cannula (18) to first pierce said seal (206) and then secondly pierce said septum (202).

The autoinjector of one of claims 21 to 28, further comprising a distal stopper (24) arranged in said dual chamber cartridge (11), and a piston rod (52), wherein, on activation of said autoinjector (10), a movement of said piston rod (52) towards the distal stopper (24) brings about a movement of said dual chamber cartridge (11) towards said cannula carriage (200).

The autoinjector of one of claims 24 to 27 and claim 29, wherein, once said septum (202) is pierced, movement of said distal stopper (24) within the dual chamber cartridge (11) is facilitated.

The autoinjector of one of claims 21 to 29, wherein the dual chamber cartridge (11) comprises a proximal (22) and a distal stopper (24) and a bypass passage (26).

The autoinjector of one of claims 21 to 30, wherein the dual chamber cartridge (11) comprises a first chamber (23) more proximally arranged than a second chamber (25), wherein the first chamber (23) is filled with a solid material (M) of said medicament and the second chamber (25) is filled with a liquid material (M') of said medicament.

The autoinjector of claims 31 and claim 32, wherein the first chamber (23) is separated from the second chamber (25) by the proximal stopper (22).

The autoinjector of one of claim 31 to 33, further comprising a piston rod (52) configured to act on said distal stopper (24) and said proximal stopper (22) and to thereby move the proximal stopper (22) such that it is arranged at the bypass passage (26) for allowing the liquid material (M') into the first chamber (23) past the proximal stopper (22) via the bypass passage (26).

A method of activating an autoinjector, the autoinjector (10) comprising a dual chamber cartridge (11) comprising first and second elements (M, M') of a medicament, with the first and second elements (M, M') each being stored in a separate chamber (23, 25) of the dual chamber cartridge (11), a cannula carriage (200) having a cannula (18) and a piston rod (52), the method comprising the steps of:
- moving the piston rod (52) proximally towards a distal stopper (24) arranged in the dual chamber cartridge (11) and thereby moving said dual chamber cartridge (11) towards the cannula carriage (200) to pierce a septum (202) of said dual chamber cartridge (11) with said cannula (18).

The method of claim 35, further comprising the step of:
allowing air to be expelled from said dual chamber cartridge (11) via said cannula (18) and then further moving the distal stopper (24) towards a proximal stopper (22) to thereby urge a second element (M') of the medicament from a second chamber (25) into a bypass passage (26) to bypass the proximal stopper (22) such that the second element (M') comes into contact with the first element (M) in the first chamber (23) arranged between the cannula carriage (200) and the proximal stopper (22).

An autoinjector, in particular according to claim 1, comprising a dual chamber cartridge (11) comprising two elements (M, M') of a medicament that are each stored in a separate chamber (2, 25) of the dual chamber cartridge (11) and the dual chamber cartridge (11) comprising a bypass passage (26) and an outer cap (34), an inner cap (32) and a cannula shield (28) covering a cannula (18) of the autoinjector (10), wherein, on activation of the autoinjector (10) said inner cap (32) is removable from the autoinjector (10) before said outer cap (34) is removed from said autoinjector (10).

The autoinjector of claim 37, wherein the autoinjector (10) is configured such that removal of the inner cap (32) simultaneously brings about a removal of the cannula shield (28).

The autoinjector of claim 37 or claim 38, wherein the inner cap (32) comprises a grip (12) projecting from the autoinjector (10).

The autoinjector of claim 39, wherein the grip (12) comprises a ring.

The autoinjector of one of claims 37 to 40, wherein removal of the inner cap (32) permits a movement of air out of the dual chamber cartridge (11).

The autoinjector of one of claims 37 to 41, wherein the inner cap (32) comprises a cannula shield holder (29) at an end of the inner cap (32) disposed opposite to a base of the inner cap (32).

The autoinjector of one of claims 37 to 42, wherein the inner cap (32) comprises inwardly facing projections (31) that engage the cannula shield (28).

The autoinjector of claim 42 and claim 43, wherein the inwardly facing projections (31) are integrally formed with the cannula shield holder (29) of the inner cap (32). The autoinjector of claim 42 and claim 43, wherein the inwardly facing projections are formed by a metal insert (30) received within the cannula shield holder (29) of the inner cap (32).

The autoinjector of one of claims 37 to 45, wherein the outer cap (34) is configured to receive a part of a cannula shield (28) of the autoinjector (10).

The autoinjector of one of claims 37 to 46, wherein an axial movement of a cannula guard (38) of the autoinjector (10) in the direction of the dual chamber cartridge (11) brings about a release of a drive mechanism of a plunger of the dual chamber cartridge (11) for dispensing a medicament (M, M') stored in the dual chamber cartridge (11).

### List of reference numerals:

- 10: autoinjector
- 11: dual chamber cartridge
- 12: handle
- 14: proximal end
- 16: distal end
- 18: cannula
- 20: pre-filled syringe
- 22: proximal stopper
- 23: first chamber
- 24: distal stopper
- 25: second chamber
- 26: bypass passage
- 28: removable cannula shield
- 29: cannula shield holder
- 30: metal pressing
- 31: projections
- 32: cap
- 34: inner cap
- 36: aperture
- 38: cannula guard
- 40: housing
- 42: lock-out spring
- 44: inner body
- 46: outer body
- 48: drive chassis
- 50: drive spring
- 52: piston rod
- 54: trigger arms
- 56: cannula guard plunger
- 58: piston rod holder
- 60: journal of spring chassis
- 62: sleeve
- 64: front end of piston rod
- 66: end of dose click arm
- 68: end of dose click ramp
- 100: cap and seal assembly
- 102: cap
- 104: seal
- 106: opening
- 108: first flange arrangement
- 109: undercut
- 110: second flange arrangement
- 111: web
- 112: rubber septum
- 200: cannula carriage
- 201: cannula carriage reception space
- 202: rubber septum
- 204: distal end
- 206: seal
- 208: detent
- 210: proximal end

## Claims

1. An autoinjector comprising a dual chamber cartridge (11) comprising two elements of a medicament (M, M') that are each stored in a separate chamber (23, 24) of the dual chamber cartridge (11) and the dual chamber cartridge (11) comprising a bypass passage (26) and the autoinjector (10) further comprising at least one security mechanism configured to secure the autoinjector against premature mixing of the elements and dispensing of said mixed elements (M, M').

2. An autoinjector, in particular according to claim 1, comprising a dual chamber cartridge (11) comprising two elements of a medicament (M, M') that are each stored in a separate chamber (23, 24) of the dual chamber cartridge (11) and the dual chamber cartridge (11) comprising a bypass passage (26), the dual chamber cartridge (11) comprising a syringe cap and seal assembly (100) covering and fixed to an outlet from the dual chamber cartridge (11).

3. The autoinjector of claim 2, wherein the cap and seal assembly (100) comprises a cap (102) and one or more seals (104) surrounded by the cap (102), in particular wherein the dual chamber cartridge (11) has an opening (106) which is covered by the cap (102) and sealed off by one of said one or more seals (104) of the cap and seal assembly (100).

4. The autoinjector of one of claims 2 to 3, wherein the cap and seal assembly (100) comprises a first flange arrangement (108) and the dual chamber cartridge (11) comprises a second flange arrangement (110) shaped complementary to the first flange arrangement (108) and being engageable by the first flange arrangement (108), in particular wherein the first flange arrangement (108) surrounds said second flange arrangement (110) and/or in particular wherein the first flange arrangement (108) is snap-fit into place at the second flange arrangement (110).

5. The autoinjector of one of claims 2 to 4, further comprising a cannula (18) held in place at the dual chamber cartridge (11) by the cap and seal assembly (100).

6. The autoinjector of claim 8 and one of claims 3 to 5, wherein the cannula (18) is held in place at the dual chamber cartridge (11) by one of said one or more seals (104) of the cap and seal assembly (100).

7. The autoinjector of one of claims 2 to 6, wherein the cap and seal assembly (100) further comprises a cannula shield (28) covering the cap and seal assembly (100), and/or wherein the dual chamber cartridge (11) further comprises a proximal (22) and a distal stopper (24), and/or wherein the dual chamber cartridge (11) comprises a first chamber (23) more proximally arranged than a second chamber (25), wherein the first chamber (23) is filled with a solid material (M) and the second chamber (25) is filled with a liquid material (M'), in particular wherein the first chamber (23) is separated from the second chamber (25) by the proximal stopper (22).

8. The autoinjector of one of claim 7, wherein the proximal stopper (22) is arranged distally of the bypass passage (26) in a storage state of the autoinjector (10), and/or wherein the proximal stopper (22) is arranged proximally of the bypass passage (26) after a dispensing operation has taken place.

9. The autoinjector of one of claims 2 to 8, further comprising a piston rod (52) configured to act on said distal stopper (24) and said proximal stopper (22) and to thereby move the proximal stopper (22) such that it is arranged at the bypass passage (26) for urging the material into the proximal chamber (23) past the proximal stopper (22) via the bypass passage (26) during a mixing operation, in particular wherein the piston rod (52) can be moved further for dispensing the medicament via the cap and seal assembly (100).

10. The autoinjector of one of claims 3 to 9, wherein the one of said one or more seals (104) of the cap and seal assembly (100) sealing off the opening (106) covers said opening (106).

11. The autoinjector of one of claims 2 to 10, further comprising a cannula shield (28) such as a rigid cannula shield (28) covering a cannula (18) of the pre-filled syringe.

12. A method of mixing a medicament in an autoinjector (10), the autoinjector (10) comprising a dual chamber cartridge (11) comprising first and second elements (M, M') of a medicament, with the first and second elements (M, M') each being stored in a separate chamber (23, 25) of the dual chamber cartridge (11), the dual chamber cartridge (11) comprising a cap and seal assembly (100) as well as a piston rod (52), the method comprising the steps of:
- moving the piston rod (52) proximally towards a distal stopper (24) and thereby moving said distal stopper (24) towards a proximal stopper (22) and reducing the distance between the distal stopper (24) and said proximal stopper (22) to thereby urge a first element (M) of the medicament from a first chamber (23) into a bypass passage (26) to bypass the proximal stopper (25) such that the first element (M) comes into contact with the second element (M') in the first chamber (25) arranged between the cap and seal assembly (100) and the proximal stopper (22).

13. An autoinjector, in particular according to claim 1, comprising a dual chamber cartridge (11) comprising two elements (M, M') of a medicament that are each stored in a separate chamber (23, 25) of the dual chamber cartridge (11), the dual chamber cartridge (11) comprising a bypass passage (26) and a rubber septum (202) and a cannula shield (28), the autoinjector (10) further comprising a cannula carriage (200) having a cannula (18) with the cannula carriage (200) being in contact with the dual chamber cartridge (11) in a storage state of the autoinjector (10) and the dual chamber cartridge (11) being moveable towards the cannula carriage (200) on activation of the autoinjector (10) in order to penetrate the septum (202) with the cannula (18).

14. The autoinjector of claim 13, wherein a distal end (204) of the cannula carriage (200) is sealed off by a seal (206), in particular wherein the seal (206) is moveably arranged in a distal cannula carriage reception space (201) relative to the cannula (18), and/or wherein the cannula carriage (200) comprises one or more detents (208) at a distal end (204) thereof, in particular wherein the cannula carriage (200) is in contact with the dual chamber cartridge (11) via the one or more detents (208) in the storage state of the autoinjector (10), and/or in particular wherein, on activation of the autoinjector (10), the dual chamber cartridge (11) is configured to overcome said one or more detents (208) such that the cannula (18) pierces the septum (202).

15. The autoinjector of one of claims 14, wherein a movement of the dual chamber cartridge (11) towards the cannula carriage (200) moves said seal (206) towards the cannula (18) to pierce said seal (206) on activation of the autoinjector (10), and/or wherein the movement of the dual chamber cartridge (11) towards the cannula carriage (200) moves said seal (206) towards the cannula (18) to first pierce said seal (206) and then secondly pierce said septum (202), and/or further comprising a distal stopper (24) arranged in said dual chamber cartridge (11), and a piston rod (52), wherein, on activation of said autoinjector (10), a movement of said piston rod (52) towards the distal stopper (24) brings about a movement of said dual chamber cartridge (11) towards said cannula carriage (200).

16. The autoinjector of one of claims 14 or 15, wherein, once said septum (202) is pierced, movement of said distal stopper (24) within the dual chamber cartridge (11) is facilitated.

17. The autoinjector of one of claims 13 to 16, wherein the dual chamber cartridge (11) comprises a proximal (22) and a distal stopper (24) and a bypass passage (26), and/or wherein the dual chamber cartridge (11) comprises a first chamber (23) more proximally arranged than a second chamber (25), wherein the first chamber (23) is filled with a solid material (M) of said medicament and the second chamber (25) is filled with a liquid material (M') of said medicament, in particular wherein the first chamber (23) is separated from the second chamber (25) by the proximal stopper (22), and/or further comprising a piston rod (52) configured to act on said distal stopper (24) and said proximal stopper (22) and to thereby move the proximal stopper (22) such that it is arranged at the bypass passage (26) for allowing the liquid material (M') into the first chamber (23) past the proximal stopper (22) via the bypass passage (26).

18. A method of activating an autoinjector, the autoinjector (10) comprising a dual chamber cartridge (11) comprising first and second elements (M, M') of a medicament, with the first and second elements (M, M') each being stored in a separate chamber (23, 25) of the dual chamber cartridge (11), a cannula carriage (200) having a cannula (18) and a piston rod (52), the method comprising the steps of:
- moving the piston rod (52) proximally towards a distal stopper (24) arranged in the dual chamber cartridge (11) and thereby moving said dual chamber cartridge (11) towards the cannula carriage (200) to pierce a septum (202) of said dual chamber cartridge (11) with said cannula (18).

19. The method of claim 18, further comprising the step of:
allowing air to be expelled from said dual chamber cartridge (11) via said cannula (18) and then further moving the distal stopper (24) towards a proximal stopper (22) to thereby urge a second element (M') of the medicament from a second chamber (25) into a bypass passage (26) to bypass the proximal stopper (22) such that the second element (M') comes into contact with the first element (M) in the first chamber (23) arranged between the cannula carriage (200) and the proximal stopper (22).

20. An autoinjector, in particular according to claim 1, comprising a dual chamber cartridge (11) comprising two elements (M, M') of a medicament that are each stored in a separate chamber (2, 25) of the dual chamber cartridge (11) and the dual chamber cartridge (11) comprising a bypass passage (26) and an outer cap (34), an inner cap (32) and a cannula shield (28) covering a cannula (18) of the autoinjector (10), wherein, on activation of the autoinjector (10) said inner cap (32) is removable from the autoinjector (10) before said outer cap (34) is removed from said autoinjector (10).

21. The autoinjector of claim 20, wherein the autoinjector (10) is configured such that removal of the inner cap (32) simultaneously brings about a removal of the cannula shield (28), and/or wherein the inner cap (32) comprises a grip (12) projecting from the autoinjector (10), in particular wherein the grip (12) comprises a ring.

22. The autoinjector of one of claims 20 or 21, wherein removal of the inner cap (32) permits a movement of air out of the dual chamber cartridge (11), and/or wherein the inner cap (32) comprises a cannula shield holder (29) at an end of the inner cap (32) disposed opposite to a base of the inner cap (32), and/or wherein the inner cap (32) comprises inwardly facing projections (31) that engage the cannula shield (28), in particular wherein the inwardly facing projections (31) are integrally formed with the cannula shield holder (29) of the inner cap (32), and/or in particular wherein the inwardly facing projections are formed by a metal insert (30) received within the cannula shield holder (29) of the inner cap (32).

23. The autoinjector of one of claims 20 to 22, wherein the outer cap (34) is configured to receive a part of a cannula shield (28) of the autoinjector (10), and/or wherein an axial movement of a cannula guard (38) of the autoinjector (10) in the direction of the dual chamber cartridge (11) brings about a release of a drive mechanism of a plunger of the dual chamber cartridge (11) for dispensing a medicament (M, M') stored in the dual chamber cartridge (11).
